(19) Europäisches Patentamt
European Patent Office
Office européen des brevets

(11) **EP 3 176 596 B1**

(12) **EUROPÄISCHE PATENTSCHRIFT**

(45) Veröffentlichungstag und Bekanntmachung des Hinweises auf die Patenterteilung:
**17.03.2021 Patentblatt 2021/11**

(51) Int Cl.:
*G01R 33/483* *(2006.01)*     *G01R 33/561* *(2006.01)*

(21) Anmeldenummer: **16193683.6**

(22) Anmeldetag: **13.10.2016**

(54) **MODIFIZIERTE TRUEFISP-SEQUENZ ZUR PARALLELEN MR-DATEN-ERFASSUNG**

MODIFIED TRUEFISP SEQUENCE FOR PARALLEL MR DATA ACQUISITION

SÉQUENCE TRUEFISP MODIFIÉE POUR COLLECTE PARALLÈLE DE DONNÉES RM

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **02.12.2015 DE 102015224054**

(43) Veröffentlichungstag der Anmeldung:
**07.06.2017 Patentblatt 2017/23**

(73) Patentinhaber:
• **Siemens Healthcare GmbH**
  **91052 Erlangen (DE)**
• **Julius-Maximilians-Universität Würzburg**
  **97070 Würzburg (DE)**

(72) Erfinder:
• **Speier, Peter**
  **91056 Erlangen (DE)**
• **Staeb, Daniel**
  **VIC 3207 Port Melbourne (AU)**

(56) Entgegenhaltungen:
**DE-A1-102014 202 358**

• **THOMAS BOYD MARTIN ET AL: "Accelerated Multiband SSFP Imaging with Controlled Aliasing in Parallel Imaging and integrated-SSFP (CAIPI-iSSFP)", PROCEEDINGS OF THE INTERNATIONAL SOCIETY FOR MAGNETIC RESONANCE IN MEDICINE, ISMRM, 23RD ANNUAL MEETING AND EXHIBITION, TORONTO, ONTARIO, CANADA, 30 MAY - 5 JUNE 2015, Nr. 3640, 15. Mai 2015 (2015-05-15), Seite 3640, XP040669316,**
• **HAACKE E M ET AL: "Steady-state free precession imaging in the presence of motion: application for improved visualization of the cerebrospinal fluid", RADIOLOGY, RADIOLOGICAL SOCIETY OF NORTH AMERICA, INC, US, Bd. 175, Nr. 2, 1. Mai 1990 (1990-05-01), Seiten 545-552, XP009030529, ISSN: 0033-8419**

**Beschreibung**

Die DE 10 2014 202 358 A1 offenbart ein Verfahren zur Ansteuerung eines Magnetresonanzbildgebungssystems zur gleichzeitigen Bildaufnahme paralleler benachbarter Schichten mit einer TrueFISP-Sequenz.

Die vorliegende Erfindung betrifft eine modifizierte TrueFISP-Sequenz, um mit dieser modifizierten TrueFISP-Sequenz gleichzeitig MR-Daten von mehreren Schichten zu erfassen. Dabei wird unter einer TrueFISP-Sequenz eine Sequenz für eine Magnetresonanzanlage verstanden, bei welcher die Gradientenmomente entlang aller drei Raumachsen ausgeglichen sind.

Die MR-Datenerfassung mit einer TrueFISP-Sequenz (True Fast Imaging with Steady State Precession) zeichnet sich durch ein sehr hohes Signal-Rausch-Verhältnis und eine hohe Geschwindigkeit aus. Zur weiteren Beschleunigung ist eine parallele Datenerfassung notwendig, bei welcher zumindest zwei Schichten gleichzeitig erfasst werden.

Bei der parallelen Datenerfassung muss das Problem gelöst werden, die empfangenen MR-Signale anteilig den verschiedenen gleichzeitig angeregten Schichten zuzuordnen. Ein Verfahren, welches diese Zuordnung oder Signaltrennung auf die verschiedenen Schichten erleichtert, ist das so genannte Caipirinha-Verfahren ("Controlled Aliasing in Parallel Imaging Results in Higher Acceleration (CAIPIRINHA) for Multi-Slice Imaging", Magn. Reson. Med. 2005; 53 (3), F.A. Breuer u.a., Seiten 684-691).

Ein weiteres bekanntes Verfahren, welches diese Zuordnung oder Signaltrennung auf die verschiedenen Schichten erleichtert, ist in US 2013/0271128 A1 beschrieben.

Wie dieses Caipirinha-Verfahren modifiziert werden kann, um es auch für TrueFISP-Sequenzen einsetzen zu können, kann beispielsweise "CAIPIRINHA accelerated SSFP imaging", Magn. Reson. Med. 2011; 65, D. Stäb u.a., Seiten 157-164 entnommen werden.

Bei diesem modifizierten Caipirinha-Verfahren wird zum einen die Phase aufeinander folgender HF-Anregungen derselben Schicht variiert. Zum anderen sind die Phasen der gleichzeitig erfolgenden HF-Anregungen der gleichzeitig zu erfassenden Schichten gegeneinander verschoben. Beim Erfassen der MR-Daten von einer Schicht S0 und einer Schicht S1 kann die Phase $P_{S0}$ der HF-Anregungen der Schicht S0 beispielsweise der folgenden Gleichung (1) genügen, während die Phase $P_{S1}$ der HF-Anregungen der Schicht S1 beispielsweise der folgenden Gleichung (2) genügen kann.

$$P_{S0} = - \; k*90° \qquad\qquad (1)$$

$$P_{S1} = + \; k*90° \qquad\qquad (2),$$

wobei k den Laufindex bezeichnet, d.h. die Phase $P_{S0}$ bzw. $P_{S1}$ ändert sich von Repetitionszeit zu Repetitionszeit um ein Phaseninkrement von -90° bzw. +90°. Die Differenz der Phaseninkremente würde demnach in diesem Fall 180° betragen.

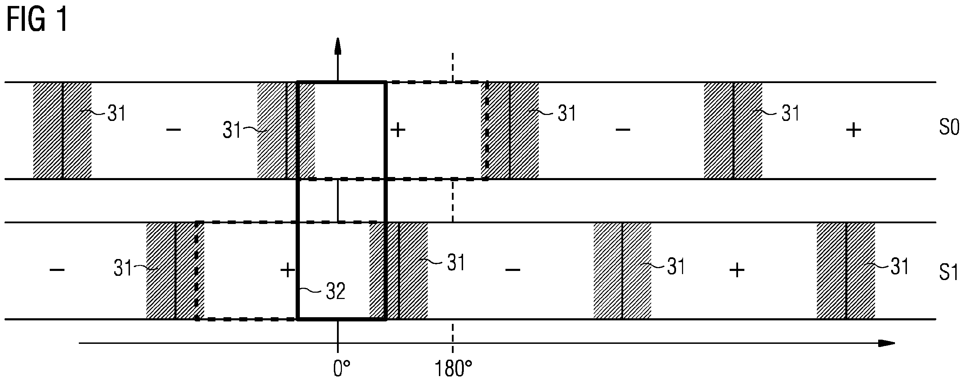

In Fig. 1 sind die Frequenzbänder der beiden gleichzeitig zu erfassenden Schichten S0 und S1 dargestellt. Die Vorzeichen innerhalb der Frequenzbänder geben an, ob die Amplitude der erfassten MR-Signale positiv oder negativ ist. Mit dem Bezugszeichen 31 sind die dunklen Bänder bezeichnet.

Man erkennt an Fig. 1, dass das modifizierte Caipirinha-Verfahren die Bandstruktur der beiden Schichten S0, S1 um jeweils 1/4 der Bandbreite verschiebt, wobei die Bandstruktur der Schicht S0 um +90° und die Bandstruktur der Schicht S1 um -90° gegenüber der Bandmitte (0°) verschoben wird. Dadurch ist die effektive Bandbreite 32 für beide Schichten S0, S1 im Vergleich zur Bandbreite für nur eine der Schichten um ungefähr 50% reduziert.

Die Erfindung stellt sich die Aufgabe, eine parallele MR-Datenerfassung mit einer TrueFISP-Sequenz bereitzustellen, wobei die effektive Bandbreite größer als bei dem vorab beschriebenen modifizierten Caipirinha-Verfahren ist.

Erfindungsgemäß wird diese Aufgabe durch ein Verfahren zum Erfassen von MR-Bilddaten nach Anspruch 1, durch eine Magnetresonanzanlage nach Anspruch 14, ein Computerprogrammprodukt nach Anspruch 16 und einen elektronisch lesbaren Datenträger nach Anspruch 17 gelöst.

Die abhängigen Ansprüche definieren bevorzugte und vorteilhafte Ausführungsformen der vorliegenden Erfindung.

Im Rahmen der vorliegenden Erfindung wird ein Verfahren zum Erfassen von MR-Signalen eines vorbestimmten Volumenabschnitts innerhalb eines Untersuchungsobjekts mittels einer Magnetresonanzanlage bereitgestellt, wobei eine Gradientenecho-Sequenz eingesetzt wird. Das Verfahren umfasst folgende Schritte:

- Schalten eines Schichtselektionsgradienten in Schichtselektionsrichtung. Dabei erzeugt der Schichtselektionsgradient ein Gradientenmoment, welches ausgeglichen ist (d.h. das Gradientenmoment über eine Repetitionszeit ist 0).

- Gleichzeitiges Anregen von zwei (oder mehr) Schichten des Volumenabschnitts mit einer HF-Anregung bzw. einem HF-Anregungspuls, welcher mehrere (insbesondere separierte) Frequenzbänder (pro anzuregender Schicht ein Frequenzband) aufweist (pro Repetitionszeit eine HF-Anregung bzw. ein HF-Anregungspuls). Dieser Multi-Band-HF-Anregungspuls kann beispielsweise aus mehreren HF-Anregungspulsen (pro Schicht und Repetitionszeit ein HF-Anregungspuls) bestehen, welche überlagert eingestrahlt werden. D.h. die eingestrahlte HF-Wellenform, mit welcher die Schichten gleichzeitig angeregt werden, entspricht einer Überlagerung der HF-Anregungspulse.

- Variieren der Phase von zu erfassenden MR-Signalen derselben Schicht von Repetitionszeit zu Repetitionszeit. Durch diesen Schritt wird die Phase der zu erfassenden MR-Signale von zumindest einer der zu erfassenden Schichten geändert, so dass diese MR-Signale der jeweiligen Schicht während einer ersten Repetitionszeit eine erste Phase aufweisen und während einer zweiten Repetitionszeit, welcher der ersten Repetitionszeit direkt nachfolgt, eine zweite Phase aufweisen, welche der ersten Phase ungleich ist. Dies gilt insbesondere für alle Repetitionszeiten, so dass die Phasen für zeitlich benachbarte Repetitionszeiten der Schicht niemals gleich sind.
  Dabei wird unter der Variation der Phase der zu erfassenden MR-Signale eine Eigenschaft der verwendeten Sequenz (und nicht beispielsweise eine Eigenschaft der aktuell abgetasteten K-Raum-Zeile) verstanden. Mit anderen Worten werden Parameter der Gradientenecho-Sequenz von Repetitionszeit zu Repetitionszeit so geändert, dass auch dann, wenn dieselbe K-Raum-Zeile zu einer ersten Repetitionszeit mit ersten Parametern der Sequenz ausgelesen wird und wenn anschließend dieselbe K-Raum-Zeile zu einer zweiten Repetitionszeit, welche der ersten Repetitionszeit direkt folgt, mit zweiten Parametern der Sequenz ausgelesen wird, sich die Phase der erfassten MR-Signale während der ersten Repetitionszeit von der Phase der erfassten MR-Signale während der zweiten Repetitionszeit unterscheidet. Wie diese Variation der Phase realisiert wird, wird im Folgenden in Form verschiedener Ausführungsformen beschrieben.
  Die Variation der Phase der MR-Signale von Repetitionszeit zu Repetitionszeit (oder von K-Raum-Zeile zu K-Raum-Zeile) ermöglicht (oder erleichtert) die Aufteilung der erfassten MR-Signal-Anteile auf die gleichzeitig zu erfassenden Schichten. Typischerweise wird durch diese Variation der Phase der MR-Signale eine Verschiebung in Phasenkodierrichtung erzeugt. Bei der Rekonstruktion kann diese Verschiebung für die jeweilige Schicht rückgängig gemacht oder berücksichtigt werden.

- Anlegen eines Zusatz-Gradienten in Schichtselektionsrichtung zusätzlich zu dem Schichtselektionsgradient. Der Zusatz-Gradient erzeugt ein Zusatz-Gradientenmoment, welches über aufeinanderfolgende Repetitionszeiten konstant ist. D.h. das Zusatz-Gradientenmoment einer ersten Repetitionszeit entspricht dem Zusatz-Gradientenmoment einer zweiten Repetitionszeit, welche der ersten Repetitionszeit direkt nachfolgt. Dies gilt insbesondere für alle Repetitionszeiten, welche zur Erfassung aller MR-Signale der gleichzeitig zu erfassenden Schichten zu erfassen sind, so dass das Zusatz-Gradientenmoment für all diese Repetitionszeiten gleich groß ist.

- Erfassen der MR-Bilddaten mit Hilfe eines Auslesegradienten.

Ohne Berücksichtigung des Zusatz-Gradienten sind die Gradientenmomente, welche von der Gradientenecho-Sequenz erzeugt werden, entlang aller drei Raumrichtungen während jeder Repetitionszeit (insbesondere von HF-Anregungspuls-Mitte bis HF-Anregungspuls-Mitte) jeweils über die Repetitionszeit hinweg ausgeglichen. Daher kann die erfindungsgemäße Gradientenecho-Sequenz als TrueFISP-Sequenz bezeichnet werden.

Durch die Erzeugung des Zusatz-Gradientenmoments kann vorteilhafterweise quasi die Larmorfrequenz für jede Schicht (genauer: für jede gleichzeitig zu erfassende Schicht) unabhängig eingestellt werden. Dadurch können die Bandstrukturen der gleichzeitig zu erfassenden Schichten (siehe Fig. 1) aneinander angepasst oder gegeneinander verschoben werden, um so die effektive Bandbreite zu vergrößern.

Anders ausgedrückt wird beim Auslesen der MR-Daten die Phase der erfassten Spins von einer Schicht nur durch den HF-Phasenzyklus in dieser Schicht und nicht durch ihre Position in der Bandstruktur bestimmt. Die Phase der erfassten Spins ist wiederum abhängig vom Unterschied der lokalen Larmorfrequenzen der Schichten. Der Unterschied der lokalen Larmorfrequenzen wird durch das Zusatz-Gradientenmoment beeinflusst, so dass durch eine entsprechende Wahl dieses Zusatz-Gradientenmoments die Bandstrukturen der gleichzeitig zu erfassenden Schichten gegeneinander verschoben werden können, um dadurch die Auslesebandbreite positiv zu beeinflussen (in der Regel zu vergrößern).

Insbesondere ist dabei der Zusatz-Gradient konstant. D.h. der Zusatz-Gradient weist während der Erfassung der MR-Signale der gleichzeitig zu erfassenden Schichten konstant dieselbe Amplitude auf.

Der konstante Zusatz-Gradient kann z.B. durch eine Shim-Einrichtung der Magnetresonanzanlage erzeugt werden, so dass die eigentliche Sequenz (und damit die Sequenzsteuerung) davon unberührt bleiben kann.

Es ist allerdings auch möglich, den Zusatz-Gradienten

- (nur) vor den HF-Anregungspulsen oder
- (nur) nach den HF-Anregungspulsen oder
- (nur) vor und nach den HF-Anregungspulsen

zu schalten. Das bedeutet, dass der Zusatz-Gradient nicht während des HF-Anregungspulses und nicht während des Auslesens der MR-Signale geschaltet wird.

Da ein dauerhaft anliegender Zusatz-Gradient die Schichtselektion und das Auslesen der MR-Signale stören kann, kann es vorteilhaft sein, den Zusatz-Gradienten nur während der Prephaser-Periode und/oder Rephaser-Periode zu schalten.

Insbesondere ist dabei der Verlauf des Zusatz-Gradienten über aufeinanderfolgende Repetitionszeiten konstant. Anders ausgedrückt entspricht der Verlauf des Zusatz-Gradienten über der Zeit während einer Repetitionszeit dem Verlauf des Zusatz-Gradienten über der Zeit während einer anderen Repetitionszeit, so dass der Verlauf des Zusatz-Gradienten über der Zeit insbesondere für alle Repetitionszeiten der gleichzeitig zu erfassenden Schichten gleich ist.

Die Einstellung eines über aufeinanderfolgende Repetitionszeiten konstanten bzw. gleichen Verlaufs des Zusatz-Gradienten erleichtert die geforderte Erzeugung des über aufeinanderfolgende Repetitionszeiten konstanten Zusatz-Gradientenmoments.

Das Zusatz-Gradientenmoment ist dabei vorteilhafterweise

- abhängig von einem Abstand zwischen den gleichzeitig anzuregenden Schichten oder
- abhängig von der Differenz des Phaseninkrements der HF-Anregungspulse der gleichzeitig anzuregenden Schichten oder
- sowohl abhängig von dem Abstand zwischen den gleichzeitig anzuregenden Schichten als auch abhängig von der Differenz des Phaseninkrements der HF-Anregungspulse der gleichzeitig anzuregenden Schichten.

Dabei gilt insbesondere, dass das Zusatz-Gradientenmoment umso größer gewählt werden sollte, je kleiner der Abstand zwischen den gleichzeitig zu erfassenden Schichten ist, und dass das Zusatz-Gradientenmoment umso größer gewählt werden sollte, desto größer die Differenz des Phaseninkrements der gleichzeitig zu erfassenden Schichten ist.

Beispielsweise kann das Zusatz-Gradientenmoment $\Delta M$ gemäß folgender Gleichung (3) bestimmt werden:

$$\Delta M = \frac{\Delta P}{\gamma \cdot d} \qquad (3)$$

Dabei entspricht $\Delta P$ der Differenz des Phaseninkrements der HF-Anregungspulse der gleichzeitig zu erfassenden Schichten, d entspricht dem Abstand zwischen den gleichzeitig zu erfassenden Schichten und $\gamma$ entspricht dem gyromagnetischen Verhältnis.

Bei zwei gleichzeitig anzuregenden bzw. zu erfassenden Schichten werden die schichtspezifischen Phasen der HF-Anregungspulse meist so gewählt, dass sich die Messfelder (Field of Views) der beiden gleichzeitig zu erfassenden Schichten (ohne Berücksichtigung des Zusatz-Gradienten) um 50 % überlappen, was einer Phaseninkrement-Differenz $\Delta P$ von 180° entspricht. In diesem Fall (d.h. bei zwei gleichzeitig zu erfassenden Schichten) wird der Zusatz-Gradient so eingestellt, dass er pro Repetitionszeit ein Zusatz-Gradientenmoment $\Delta M$ erzeugt, welches gemäß der folgenden Gleichung (4) berechnet werden kann.

$$\Delta M = \frac{180°}{\gamma \cdot d} \qquad (4)$$

Gemäß einer bevorzugten erfindungsgemäßen Ausführungsform gehorcht die Phase $P_0(k)$ der HF-Anregungspulse einer ersten Schicht der folgenden Gleichung (5), während die Phase $P_1(k)$ der HF-Anregungspulse einer zweiten Schicht, deren MR-Daten gleichzeitig mit den MR-Daten der ersten Schicht zu erfassen sind, der folgenden Gleichung (6) gehorcht.

$$P_0(k) = -k * 90° - k * \Phi_G + \Phi_{C0} \qquad (5)$$

$$P_1(k) = +k * 90° - k * \Phi_G + \Phi_{C1} \qquad (6)$$

k ist dabei ein Laufindex, welcher bei 0 beginnt. Die Variable $\Phi_{C0}$ bezeichnet eine beliebige konstante Phase bzw. Phasenkonstante in Schicht S0, und die Variable $\Phi_{C1}$ bezeichnet eine beliebige konstante Phase bzw. Phasenkonstante in Schicht S1. Die beiden Variablen können gleich oder unterschiedlich oder auch einzeln oder beide gleich Null sein. Das Phaseninkrement $\Phi_G$ wird gemäß der folgenden Gleichung (7) berechnet.

$$\Phi_G = 90° \cdot \frac{(d0 + d1)}{(d0 - d1)} \qquad (7)$$

Dabei gibt d0 den Abstand der ersten Schicht in einer vorbestimmten Richtung vom Isozentrum an, während d1 den Abstand der zweiten Schicht in der vorbestimmten Richtung vom Isozentrum angibt. Wenn beide Schichten denselben Abstand vom Isozentrum aufweisen, gilt d0 = -d1 und somit $\Phi_G = 0°$. D.h. in diesem Fall ist kein Phaseninkrement zu berücksichtigen.

Beispielsweise gilt für k=3 $P_0(3) = 90° - 3*\Phi_G$ und $P_1(3) = -90° - 3*\Phi_G$. Die Phaseninkrement-Differenz beträgt 180°, da sich der Term (- k * $\Phi_G$) bei der Differenzbildung herauskürzt.

Die Phase $\Phi_E$ der Empfangsspule(n) wird dabei vorteilhafterweise entsprechend der folgenden Gleichung (8) gewählt.

$$\Phi_E = X(k) + k * \Phi0 - k * \Phi_G \qquad (8)$$

Dabei gilt X(k) = 0°, wenn k ungerade ist und sonst gilt X(k) = 180°. Der Phasenwert $\Phi0$ wird abhängig vom Rekonstruktionsalgorithmus gewählt. Ein beispielhafter Wert für $\Phi0$ ist 90°.

Das Variieren der Phase der zu erfassenden MR-Signale wird vorteilhafterweise für jede der gleichzeitig zu erfassenden Schichten durchgeführt. Dabei sind beispielsweise die Phasen der zu erfassenden MR-Signale während zwei zeitlich aufeinanderfolgenden Repetitionszeiten niemals gleich, was insbesondere für jede Schicht gilt.

Das Variieren der Phase der zu erfassenden MR-Signale kann auf folgende Weisen erzielt werden:

- Ein weiterer Gradient wird vor und nach dem jeweiligen HF-Anregungspuls in Schichtselektionsrichtung angelegt. Dabei ist das weitere Gradientenmoment, welches von dem weiteren Gradient vor dem jeweiligen HF-Anregungspuls erzeugt wird, gleich dem weiteren Gradientenmoment, welches von dem weiteren Gradient nach dem jeweiligen HF-Anregungspuls erzeugt wird. Im Gegensatz zum Zusatz-Gradientenmoment variiert das weitere Gradientenmoment von Repetitionszeit zu Repetitionszeit, wodurch die Variation der Phase der zu erfassenden MR-Signale erzielt wird. Dieses Vorgehen ist als "blipped Caipirinha" bekannt (siehe z.B. US 2013/0271128 A1).

- Die Phase, mit welcher dieselbe Schicht durch die HF-Anregungspulse angeregt wird, wird variiert. Dieses Vorgehen ist als Caipirinha-Verfahren bekannt.

Beim Einsatz des blipped Caipirinha-Verfahrens ändert sich vorteilhafterweise die Polarität des von dem weiteren Gradient erzeugten weiteren Gradientenmoments von Repetitionszeit zu Repetitionszeit, wobei der Betrag des pro Repetitionszeit erzeugten weiteren Gradientenmoments konstant bleibt.

Dieser Polaritätswechsel des weiteren Gradientenmoments erfordert eine gute Kompensation von Wirbelströmen, damit sich die Wirbelstrom-Effekte nicht von Repetitionszeit zu Repetitionszeit aufaddieren und dadurch den Gleichgewichtszustand (steady state) der Spins stören, wodurch Bild-Artefakte erzeugt werden.

Vorteilhafterweise wird beim Einsatz des normalen Caipirinha-Verfahrens (zum Variieren der Phase der zu erfassenden MR-Signale) bei jeder Schicht die Phase von zwei aufeinanderfolgenden HF-Anregungspulsen derselben Schicht verändert. Dabei sind beispielsweise die Phasen von zwei zeitlich aufeinanderfolgenden HF-Anregungspulsen derselben Schicht niemals gleich, was insbesondere für jede gleichzeitig zu erfassende Schicht gilt.

Es sei darauf hingewiesen, dass die beiden Vorgehen zur Variation der Phase der zu erfassenden MR-Signale kombiniert werden können. Um beispielsweise eine vorbestimmte Phaseninkrement-Differenz zwischen Schichten zu erzielen, kann ein Anteil dieser Differenz durch das eine Vorgehen (z.B. Caipirinha) und der restliche andere Anteil dieser Differenz durch das andere Vorgehen (z.B. blipped Caipirinha) erzeugt werden.

Die vorliegende Erfindung kann auch eingesetzt werden, um gleichzeitig MR-Signale von mehr als zwei Schichten zu erfassen. Da das erfindungsgemäße Zusatz-Gradientenmoment zu einer Frequenzverschiebung führt, welche proportional vom Abstand der Schichten abhängt, muss die durch das entsprechende Caipirinha-Verfahren erzeugte Phasenverschiebung den Abstand der jeweils benachbarten Schichten berücksichtigen. Mit anderen Worten sollte die durch das entsprechende Caipirinha-Verfahren erzeugte Phasenverschiebung zwischen den jeweils benachbarten Schichten proportional zu dem Abstand dieser Schichten sein. Mit anderen Worten gilt folgende Beziehung (9).

$$\Delta PV_{x,y} \sim \Delta FOV_{x,y} \sim d_{x,y} \qquad (9).$$

Dabei entspricht $\Delta PV_{x,y}$ der durch das Caipirinha-Verfahren erzeugten Phasenverschiebung zwischen den direkt miteinander benachbarten Schichten x und y, $\Delta FOV_{x,y}$ entspricht der entsprechenden Verschiebung der Messfelder der beiden direkt miteinander benachbarten Schichten x und y und $d_{x,y}$ entspricht dem Abstand zwischen diesen beiden Schichten x und y.

Wenn beispielsweise MR-Signale von drei Schichten S1, S2, S3 gleichzeitig erfasst werden sollen und der Abstand $d_{S1,S2}$ zwischen den direkt benachbarten Schichten doppelt so groß ist wie der Abstand $d_{S2,S3}$ zwischen den beiden Schichten S2 und S3, dann sollte auch die Phasenverschiebung $\Delta PV_{S1,S2}$ und die Verschiebung der Messfelder $\Delta FOV_{S1,S2}$ zwischen den beiden Schichten S1 und S2 doppelt so groß sein, wie die Phasenverschiebung $\Delta PV_{S2,S3}$ und die Verschiebung der Messfelder $\Delta FOV_{S2,S3}$ zwischen den beiden Schichten S2 und S3.

Bei drei gleichzeitig zu erfassenden Schichten könnten beispielsweise Phaseninkrement von -120°, 0° und 120° für die HF-Anregungen der drei Schichten verwendet werden, welche äquidistant angeordnet sind. In diesem Fall würden die Messfelder benachbarter Schichten um je 1/3 eines Messfelds gegeneinander verschoben sein.

Ab vier gleichzeitig zu erfassenden Schichten können die Phaseninkrement für die HF-Anregungen der jeweiligen Schichten beispielsweise 0°, 90°, 180°, 270°, usw. oder 0°, 180°, 270°, 90°, usw. betragen.

Man kann sich die vorliegende Erfindung auch als eine Verbesserung des modifizierten Caipirinha-Verfahrens vorstellen (siehe "CAIPIRINHA accelerated SSFP imaging", Magn. Reson. Med. 2011; 65, D. Stäb u.a., Seiten 157-164). Dabei wird diesem modifizierten Caipirinha-Verfahren der Zusatz-Gradient hinzugefügt, um die effektive Auslesebandbreite zu vergrößern.

Im Rahmen der vorliegenden Erfindung wird auch eine Magnetresonanzanlage zum Erfassen von MR-Signalen eines Volumenabschnitts eines Untersuchungsobjekts unter Verwendung einer Gradienten-Echosequenz bereitgestellt. Dabei wird unter der Gradientenecho-Sequenz eine Sequenz verstanden, welche ohne Berücksichtigung des Zusatz-Gradienten Gradientenmomente erzeugt, die entlang aller drei Raumrichtungen jeweils über eine Repetitionszeit hinweg ausgeglichen sind.

Die Magnetresonanzanlage umfasst einen Grundfeldmagneten, ein Gradientenfeldsystem, eine oder meist mehrere HF-Antennen und eine Steuereinrichtung zur Ansteuerung des Gradientenfeldsystems und der HF-Antenne(n), zum Empfang von von der mindestens einen HF-Antenne aufgenommenen MR-Signalen und zur Auswertung dieser MR-Signale. Die Magnetresonanzanlage ist ausgestaltet, um mit dem Gradientenfeldsystem (3) einen Schichtselektionsgradient in Schichtselektionsrichtung zu schalten, welcher ein ausgeglichenes Gradientenmoment erzeugt,
um mit der mindestens einen HF-Antenne gleichzeitig mehrere Schichten des Volumenabschnitts mit Hilfe einer HF-Anregung bzw. eines HF-Anregungspulses, welcher mit der Repetitionszeit wiederholt wird, anzuregen,
um mit der Steuereinrichtung die Phase von zu erfassenden MR-Signalen einer selben der Schichten von Repetitionszeit zu Repetitionszeit zu variieren,
um mit dem Gradientenfeldsystem einen Zusatz-Gradient in Schichtselektionsrichtung zusätzlich zu dem Schichtselektionsgradient anzulegen, und
um mit dem Gradientenfeldsystem einen Auslesegradient zu schalten und mittels des Auslesegradienten und der mindestens einen HF-Antenne die MR-Signale zu erfassen.

Dabei erzeugt der Zusatz-Gradient ein Zusatz-Gradientenmoment, welches über aufeinanderfolgende Repetitionszeiten konstant ist. Das Zusatz-Gradientenmoment verletzt die Bedingung, dass die Gradientenmomente der Gradientenecho-Sequenz entlang aller drei Raumrichtungen jeweils über die Repetitionszeit hinweg ausgeglichen sind.

Die Vorteile der erfindungsgemäßen Magnetresonanzanlage entsprechen dabei im Wesentlichen den Vorteilen des erfindungsgemäßen Verfahrens, welche vorab im Detail ausgeführt worden sind, so dass hier auf eine Wiederholung verzichtet wird.

Des Weiteren beschreibt die vorliegende Erfindung ein Computerprogrammprodukt, insbesondere ein Computerprogramm oder eine Software, welche man in einen Speicher einer programmierbaren Steuerung bzw. einer Recheneinheit einer Magnetresonanzanlage laden kann. Mit diesem Computerprogrammprodukt können alle oder verschiedene vorab beschriebene Ausführungsformen des erfindungsgemäßen Verfahrens ausgeführt werden, wenn das Computerprogrammprodukt in der Steuerung oder Steuereinrichtung der Magnetresonanzanlage läuft. Dabei benötigt das Computerprogrammprodukt eventuell Programmmittel, z.B. Bibliotheken und Hilfsfunktionen, um die entsprechenden Ausführungsformen der Verfahren zu realisieren. Mit anderen Worten soll mit dem auf das Computerprogrammprodukt gerichteten Anspruch insbesondere ein Computerprogramm oder eine Software unter Schutz gestellt werden, mit welcher eine der oben beschriebenen Ausführungsformen des erfindungsgemäßen Verfahrens ausgeführt werden kann bzw. welche diese Ausführungsform ausführt. Dabei kann es sich bei der Software um einen Quellcode (z.B. C++), der noch compiliert (übersetzt) und gebunden oder der nur interpretiert werden muss, oder um einen ausführbaren Softwarecode handeln, der zur Ausführung nur noch in die entsprechende Recheneinheit bzw. Steuereinrichtung zu laden ist.

Schließlich offenbart die vorliegende Erfindung einen elektronisch lesbaren Datenträger, z.B. eine DVD, ein Magnetband, eine Festplatte oder einen USB-Stick, auf welchem elektronisch lesbare Steuerinformationen, insbesondere Software (vgl. oben), gespeichert ist. Wenn diese Steuerinformationen (Software) von dem Datenträger gelesen und in eine Steuereinrichtung bzw. Recheneinheit einer Magnetresonanzanlage gespeichert werden, können alle erfindungsgemäßen Ausführungsformen des vorab beschriebenen Verfahrens durchgeführt werden.

Die vorliegende Erfindung wird insbesondere zur Verbesserung von Caipirinha-Verfahren bei paralleler MR-Datenerfassung eingesetzt. Selbstverständlich ist die vorliegende Erfindung nicht auf diesen bevorzugten Anwendungsbereich eingeschränkt, da die vorliegende Erfindung beispielsweise auch bei Varianten des Caipirinha-Verfahrens (z.B. blipped Caipirinha) eingesetzt werden kann.

Im Folgenden wird die vorliegende Erfindung anhand bevorzugter erfindungsgemäßer Ausführungsformen mit Bezug zu den Figuren im Detail beschrieben.

In Fig. 1 ist das von der Erfindung zu lösende Problem in Form von gegeneinander verschobenen Frequenzbändern von zwei gleichzeitig zu erfassenden Schichten dargestellt.

In Fig. 2 ist die Grundidee der Erfindung dargestellt.

In Fig. 3 ist eine erste Variante zur Erzeugung des erfindungsgemäßen Zusatz-Gradientenmoments dargestellt.

In Fig. 4 ist eine zweite Variante zur Erzeugung des erfindungsgemäßen Zusatz-Gradientenmoments dargestellt.

In Fig. 5 ist eine dritte Variante zur Erzeugung des erfindungsgemäßen Zusatz-Gradientenmoments dargestellt.

In Fig. 6 ist ein weiteres von der Erfindung zu lösendes Problem dargestellt.

In Fig. 7 ist schematisch eine erfindungsgemäße Magnetresonanzanlage dargestellt.

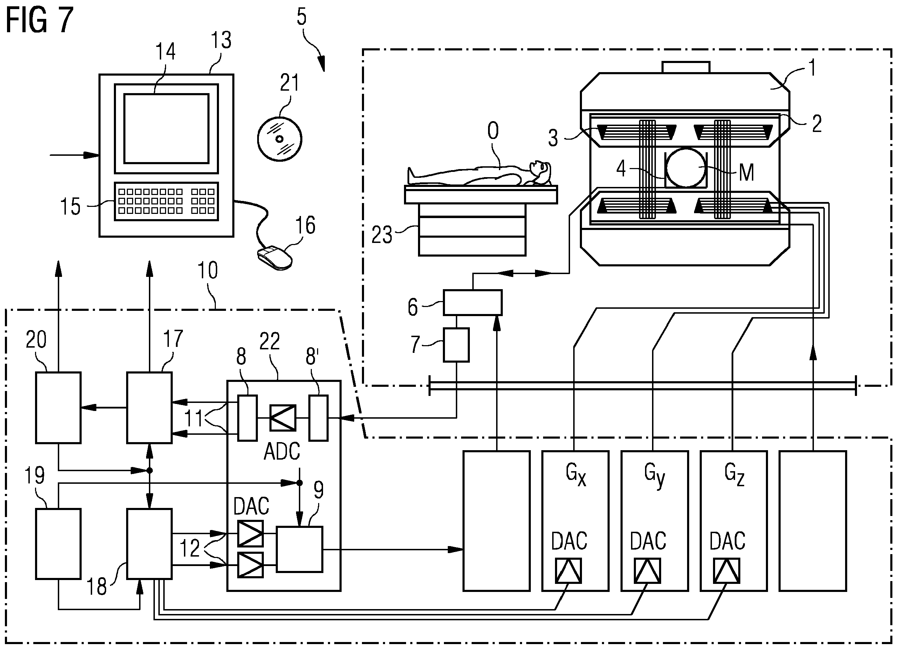

Fig. 7 ist eine schematische Darstellung einer erfindungsgemäßen Magnetresonanzanlage 5 (eines Magnetresonanz-Bildgebungs- bzw. Kernspintomographiegeräts). Dabei erzeugt ein Grundfeldmagnet 1 ein zeitlich konstantes starkes Magnetfeld zur Polarisation bzw. Ausrichtung der Kernspins in einem Untersuchungsbereich eines Objekts O, wie z.B. eines zu untersuchenden Teils eines menschlichen Körpers, welcher auf einem Tisch 23 liegend in der Magnetresonanzanlage 5 untersucht wird. Die für die Kernspinresonanzmessung erforderliche hohe Homogenität des Grundmagnetfelds ist in einem typischerweise kugelförmigen Messvolumen M definiert, in welchem der zu untersuchende Volumenabschnitt des menschlichen Körpers angeordnet ist. Zur Unterstützung der Homogenitätsanforderungen und insbesondere zur Eliminierung zeitlich invariabler Einflüsse werden an geeigneter Stelle so genannte Shim-Bleche aus ferromagnetischem Material angebracht. Zeitlich variable Einflüsse werden durch Shim-Spulen 2 eliminiert.

In den Grundfeldmagneten 1 ist ein zylinderförmiges Gradientenfeldsystem bzw. Gradientenfeldsystem 3 eingesetzt, welches aus drei Teilwicklungen besteht. Jede Teilwicklung wird von einem Verstärker mit Strom zur Erzeugung eines linearen (auch zeitlich veränderbaren) Gradientenfeldes in die jeweilige Richtung des kartesischen Koordinatensystems versorgt. Die erste Teilwicklung des Gradientenfeldsystems 3 erzeugt dabei einen Gradienten $G_x$ in x-Richtung, die zweite Teilwicklung einen Gradienten $G_y$ in y-Richtung und die dritte Teilwicklung einen Gradienten $G_z$ in z-Richtung. Der Verstärker umfasst einen Digital-Analog-Wandler, welcher von einer Sequenzsteuerung 18 zum zeitrichtigen Erzeugen von Gradientenpulsen angesteuert wird.

Innerhalb des Gradientenfeldsystems 3 befindet sich eine (oder mehrere) Hochfrequenzantennen 4, welche die von einem Hochfrequenzleistungsverstärker abgegebenen Hochfrequenzpulse in ein magnetisches Wechselfeld zur Anregung der Kerne und Ausrichtung der Kernspins des zu untersuchenden Objekts O bzw. des zu untersuchenden Bereiches des Objekts O umsetzen. Jede Hochfrequenzantenne 4 besteht aus einer oder mehreren HF-Sendespulen und einer oder mehreren HF-Empfangsspulen in Form einer ringförmigen vorzugsweise linearen oder matrixförmigen Anordnung von Komponentenspulen. Von den HF-Empfangsspulen der jeweiligen Hochfrequenzantenne 4 wird auch das von den präzedierenden Kernspins ausgehende Wechselfeld, d.h. in der Regel die von einer Pulssequenz aus einem oder mehreren Hochfrequenzpulsen und einem oder mehreren Gradientenpulsen hervorgerufenen Kernspinechosignale, in eine Spannung (Messsignal) umgesetzt, welche über einen Verstärker 7 einem Hochfrequenz-Empfangskanal 8 eines Hochfrequenzsystems 22 zugeführt wird. Das Hochfrequenzsystem 22, welches Teil einer Steuereinrichtung 10 der Magnetresonanzanlage 5 ist, umfasst weiterhin einen Sendekanal 9, in welchem die Hochfrequenzpulse für die Anregung der magnetischen Kernresonanz erzeugt werden. Dabei werden die jeweiligen Hochfrequenzpulse aufgrund einer vom Anlagerechner 20 vorgegebenen Pulssequenz in der Sequenzsteuerung 18 digital als Folge komplexer Zahlen dargestellt. Diese Zahlenfolge wird als Real- und als Imaginärteil über jeweils einen Eingang 12 einem Digital-Analog-Wandler im Hochfrequenzsystem 22 und von diesem einem Sendekanal 9 zugeführt. Im Sendekanal 9 werden die Pulssequenzen einem Hochfrequenz-Trägersignal aufmoduliert, dessen Basisfrequenz der Resonanzfrequenz der Kernspins im Messvolumen entspricht.

Die Umschaltung von Sende- auf Empfangsbetrieb erfolgt über eine Sende-/Empfangsweiche 6. Die HF-Sendespulen der Hochfrequenzantenne(n) 4 strahlt/en die Hochfrequenzpulse zur Anregung der Kernspins in das Messvolumen M ein und resultierende Echosignale werden über die HF-Empfangsspule(n) abgetastet. Die entsprechend gewonnenen Kernresonanzsignale werden im Empfangskanal 8' (erster Demodulator) des Hochfrequenzsystems 22 phasenempfindlich auf eine Zwischenfrequenz demoduliert, im Analog-Digital-Wandler (ADC) digitalisiert und über den Ausgang 11 ausgegeben. Dieses Signal wird noch auf die Frequenz 0 demoduliert. Die Demodulation auf die Frequenz 0 und die Trennung in Real- und Imaginärteil findet nach der Digitalisierung in der digitalen Domäne in einem zweiten Demodulator 8 statt. Durch einen Bildrechner 17 wird aus den dergestalt über einen Ausgang 11 gewonnenen Messdaten ein MR-Bild rekonstruiert. Die Verwaltung der Messdaten, der Bilddaten und der Steuerprogramme erfolgt über den Anlagenrechner 20. Aufgrund einer Vorgabe mit Steuerprogrammen kontrolliert die Sequenzsteuerung 18 die Erzeugung der jeweils gewünschten Pulssequenzen und das entsprechende Abtasten des k-Raumes. Insbesondere steuert die Sequenzsteuerung 18 dabei das zeitrichtige Schalten der Gradienten, das Aussenden der Hochfrequenzpulse mit definierter Phasenamplitude sowie den Empfang der Kernresonanzsignale. Die Zeitbasis für das Hochfrequenzsystem 22 und die Sequenzsteuerung 18 wird von einem Synthesizer 19 zur Verfügung gestellt. Die Auswahl entsprechender Steuerprogramme zur Erzeugung eines MR-Bildes, welche z.B. auf einer DVD 21 gespeichert sind, sowie die Darstellung des erzeugten MR-Bildes erfolgt über ein Terminal 13, welches eine Tastatur 15, eine Maus 16 und einen Bildschirm 14 umfasst.

Bei der vorliegenden Erfindung ist die Sequenzsteuerung 18 ausgestaltet, um auch den Zusatz-Gradient zu schalten.

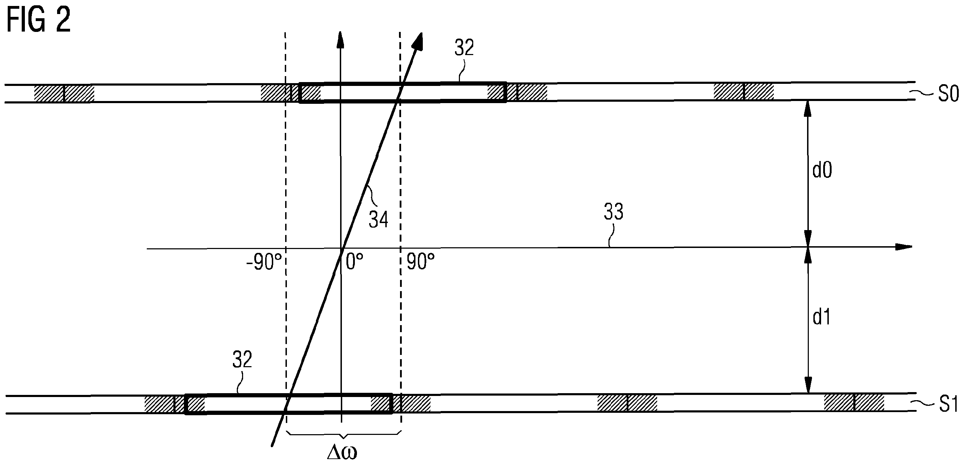

In Fig. 2 ist die erfinderische Idee dargestellt. Bei der in Fig. 2 dargestellten Situation liegt das Isozentrum des Grundmagnetfelds in der Mitte zwischen den beiden Schichten S0, S1, deren MR-Signale gleichzeitig erfasst werden sollen, so dass die beiden Schichten S0, S1 vom Betrag her denselben Abstand vom Isozentrum 32 aufweisen. Beispielsweise aufgrund eines modifizierten Caipirinha-Verfahrens sind die Frequenzbänder 32 der beiden Schichten S0, S1 um 180° gegeneinander verschoben. Diese Verschiebung soll durch eine Anpassung der lokalen Larmorfrequenz 34 (d.h. durch eine entsprechende Einstellung der Larmorfrequenz der Schichten S0, S1) quasi aufgehoben werden. Dadurch würden die Frequenzbänder 32 der beiden Schichten S0, S1 quasi übereinander liegen, wodurch die effektive Auslesebandbreite einen maximalen Wert aufweisen würde.

Zur Anpassung der lokalen Larmorfrequenz 34 wird erfindungsgemäß ein Zusatz-Gradientenmoment $\Delta M$ erzeugt, mit welchem die Frequenzband-Verschiebung $\Delta\omega$ zwischen den Frequenzbändern 32 der Schichten S0, S1 aufgehoben wird. Anders ausgedrückt wird durch das Zusatz-Gradientenmoment $\Delta M$ die Larmorfrequenz umso stärker erhöht (verkleinert), je weiter der Abstand in einer vorbestimmten Richtung (entgegen der vorbestimmten Richtung) vom Isozentrum 33 ist. Dabei ist die vorbestimmte Richtung in Fig. 2 nach oben (von Schicht S1 nach Schicht S0) gerichtet.

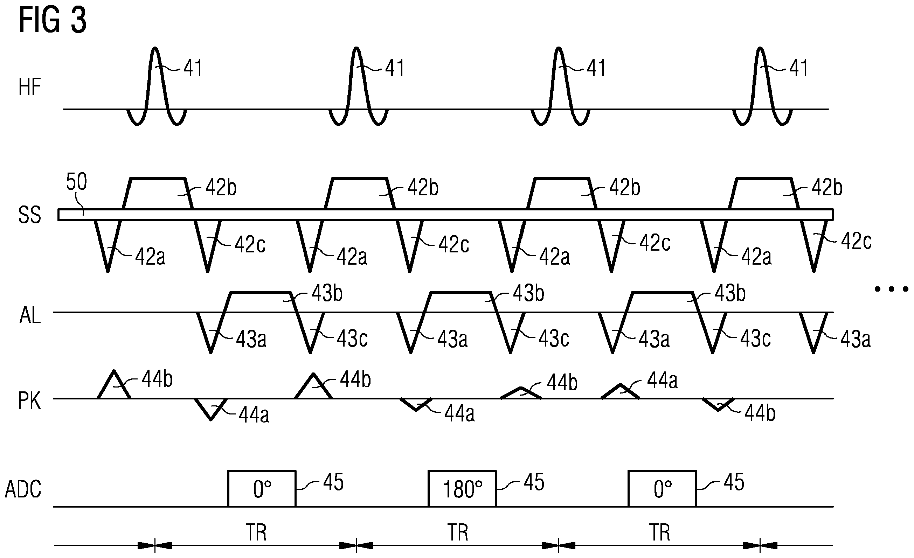

In Fig. 3 ist eine erste erfindungsgemäße Ausführungsform zur Erzeugung des Zusatz-Gradientenmoments $\Delta M$ dargestellt.

Die in Fig. 3 für eine von mehreren gleichzeitig zu erfassenden Schichten dargestellte Gradientenecho-Sequenz umfasst einen HF-Anregungspuls 41, welcher zeitgleich mit einem anliegenden Schichtselektionsgradienten 42 eingestrahlt wird. Nach dem HF-Anregungspuls 41 werden ein Phasenkodiergradient 44 und ein Auslesegradient 43 geschaltet. Während der Auslesegradient 43 anliegt, erfolgt das Auslesen der MR-Signale während einer bestimmten Zeitspanne 45. Die in der jeweiligen Zeitspanne 45 angegebene Gradzahl (0° oder 180°) verdeutlicht die entsprechende Phasenlage der MR-Signale.

Das erfindungsgemäße Zusatz-Gradientenmoment wird bei der in Fig. 3 dargestellten Ausführungsform durch einen Zusatz-Gradient 50 erzeugt, welcher über der Zeit konstant anliegt. Da es sich bei der dargestellten erfindungsgemäßen Gradientenecho-Sequenz um eine ausgeglichene Sequenz handelt, ist das von den Gradienten 42, 43, 44 in allen drei Raumrichtungen (d.h. in Schichtselektionsrichtung SS, Ausleserichtung AL und Phasenkodierrichtung PK) pro Repetitionszeit TR erzeugte Gradientenmoment Null. Diese Bedingung wird durch den erfindungsgemäßen Zusatz-Gradient 50 verletzt.

Genauer gesagt entspricht das von den Gradientenanteilen 42a und 42c des Schichtselektionsgradienten 42 erzeugte Gradientenmoment vom Betrag her dem von dem Gradientenanteil 42b des Schichtselektionsgradienten erzeugten Gradientenmoment. In ähnlicher Weise entspricht das von den Gradientenanteilen 43a und 43c des Auslesegradienten 43 erzeugte Gradientenmoment vom Betrag her dem von dem Gradientenanteil 43b erzeugten Gradientenmoment. Die Gradientenmomente, welche von den Gradientenanteilen 44a, 44b des Phasenkodiergradienten 44 erzeugt werden, sind ebenfalls vom Betrag her gleich.

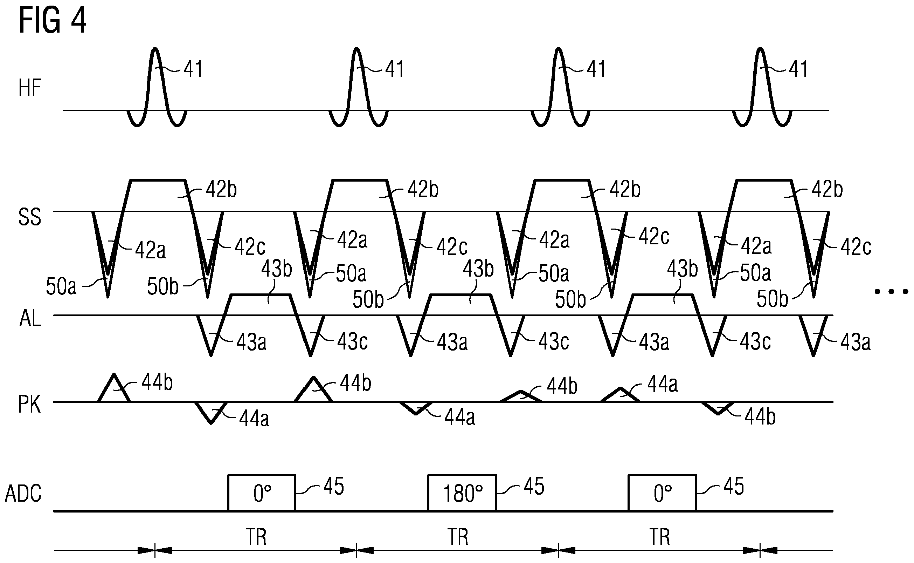

In Fig. 4 ist eine bevorzugte erfindungsgemäße Ausführungsform zur Erzeugung des Zusatz-Gradientenmoments dargestellt.

Im Unterschied zu Fig. 3 wird das Zusatz-Gradientenmoment bei der in Fig. 4 dargestellten Ausführungsform durch einen Zusatz-Gradienten 50 erzeugt, welcher nur in den Zeitspannen vor und nach dem HF-Anregungspuls 41 anliegt und daher nicht in der Zeitspanne anliegt, während welcher der HF-Anregungspuls 41 eingestrahlt wird. Darüber hinaus liegt der Zusatz-Gradient 50 auch nicht in der Zeitspanne 45 an, während welcher die MR-Signale ausgelesen werden. Anders ausgedrückt wird der Zusatz-Gradient 50 nur in der sogenannten Prephaser-Phase und in der sogenannten Rephaser-Phase des Schichtselektionsgradienten 42 angelegt. Mit anderen Worten wird bei dieser erfindungsgemäßen Ausführungsform der Zusatz-Gradient in zwei Anteile 50a, 50b aufgeteilt, wobei der eine Anteil 50a dem Prephaser-Anteil 42a des Schichtselektionsgradienten 42 und der andere Anteil 50b dem Rephaser-Anteil 42c des Schichtselektionsgradienten 42 überlagert wird. Der Vorteil dieser Ausführungsform ist, dass die Schichtselektion durch den HF-Anregungspuls 41 und das Auslesen der MR-Signale nicht durch den Zusatz-Gradient 50 gestört werden.

Es sei darauf hingewiesen, dass das von dem Zusatz-Gradient 50 erzeugte Zusatz-Gradientenmoment für jede Repetitionszeit TR gleich groß ist. Um während jeder Repetitionszeit TR dasselbe Zusatz-Gradientenmoment zu erzeugen, muss der (Verlauf des) Zusatz-Gradient(en) nicht während jeder Repetitionszeit konstant oder gleich sein. Es ist durchaus möglich, die Bedingung, dass das Zusatz-Gradientenmoment während jeder Repetitionszeit TR gleich sein muss, mit verschiedenen Zusatz-Gradienten innerhalb der jeweiligen Repetitionszeit TR zu erfüllen. Allerdings ist bei der in Fig. 4 dargestellten Ausführungsform auch der (Verlauf des) Zusatz-Gradient(en) für alle Repetitionszeiten TR gleich.

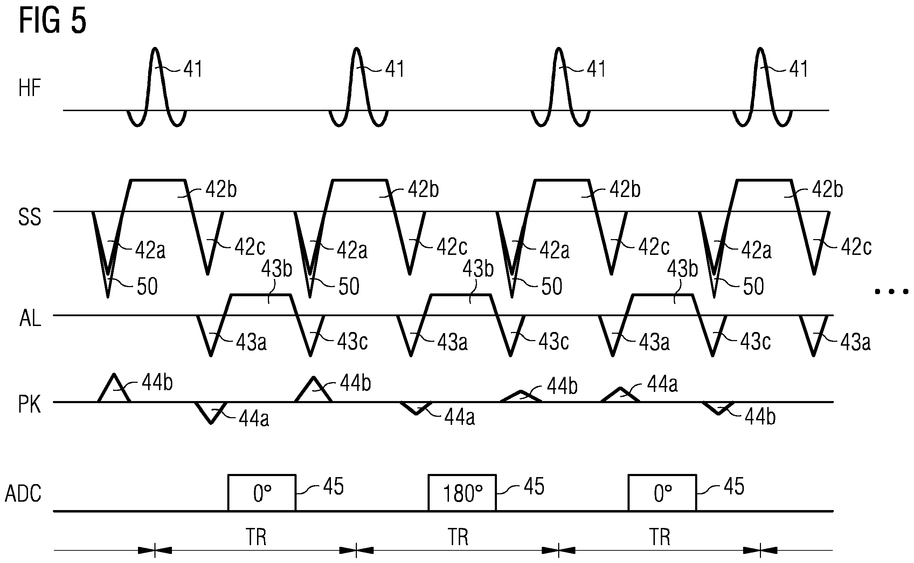

In Fig. 5 ist eine weitere erfindungsgemäße Ausführungsform dargestellt.

Im Unterschied zu der in Fig. 4 dargestellten Ausführungsform wird der Zusatz-Gradient 50 bei der in Fig. 5 dargestellten Ausführungsform nur unmittelbar vor dem HF-Anregungspuls 41 geschaltet. D.h. der Zusatz-Gradient 50 wird bei dieser Ausführungsform weder während des HF-Anregungspulses 41 noch während des Auslesens der MR-Signale noch direkt nach dem HF-Anregungspuls 41 geschaltet. Anders ausgedrückt wird der Zusatz-Gradient 50 nur in der sogenannten Prephaser-Phase des Schichtselektionsgradienten 42 geschaltet, so dass der Zusatz-Gradient 50 nur dem Prephaser-Anteil 42a des Schichtselektionsgradienten 42 überlagert wird.

Auch bei dieser Ausführungsform ist das pro Repetitionszeit TR erzeugte Zusatz-Gradientenmoment über alle Repetitionszeiten TR konstant. Obwohl es nicht notwendig ist (vergleiche Erläuterung zu der in Fig. 4 dargestellten Ausführungsform), ist daher auch der (Verlauf des) Zusatz-Gradient(en) 50 für alle Repetitionszeiten TR gleich.

Gemäß einer weiteren (aber nicht dargestellten) erfindungsgemäßen Ausführungsform kann der Zusatz-Gradient 50 auch nur unmittelbar nach dem HF-Anregungspuls 41 geschaltet werden. D.h. der Zusatz-Gradient 50 wird bei dieser Ausführungsform weder während des HF-Anregungspulses 41 noch während des Auslesens der MR-Signale noch direkt vor dem HF-Anregungspuls 41 geschaltet. Anders ausgedrückt wird der Zusatz-Gradient 50 nur in der sogenannten Rephaser-Phase des Schichtselektionsgradienten 42 geschaltet, so dass der Zusatz-Gradient 50 nur dem Rephaser-Anteil 42c des Schichtselektionsgradienten 42 überlagert wird.

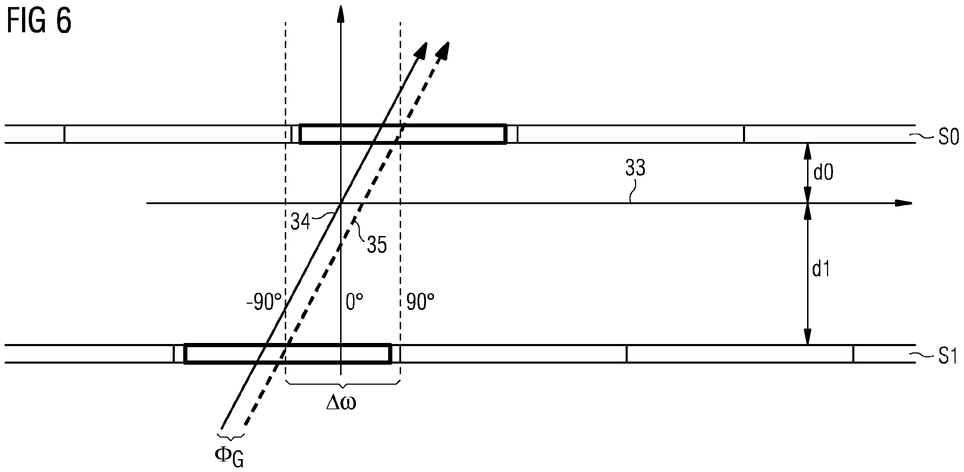

In Fig. 6 ist eine Eigenart der vorliegenden Erfindung dargestellt, welche auftritt, wenn sich das Isozentrum 33 nicht in der Mitte der beiden gleichzeitig zu erfassenden Schichten S0, S1 befindet, wie es im Allgemeinen der Fall ist. In diesem Fall akkumulieren die erfassten MR-Signale eine unerwünschte Phase $\Phi_G$ pro Repetitionszeit TR.

Diese unerwünschte Phasenakkumulation $\Phi_{G\,pro\,TR}$ kann vermieden werden, indem diese unerwünschte Phasenakkumulation bestimmt und dann sowohl bei der Bestimmung der Phase der Anregungspulse als auch bei der Bestimmung der Phase des Empfängers berücksichtigt wird. Als Ergebnis weisen die erfassten MR-Signale die unerwünschte Phasenakkumulation $\Phi_G$ nicht mehr auf.

## Patentansprüche

1. Verfahren zum Erfassen von MR-Signalen eines vorbestimmten Volumenabschnitts (24) innerhalb eines Untersuchungsobjekts (O) mittels einer Magnetresonanzanlage (5) unter Einsatz einer Gradientenecho-Sequenz, folgende Schritte umfassend:

   Schalten eines Schichtselektionsgradienten (42) in Schichtselektionsrichtung (SS), welcher ein ausgeglichenes Gradientenmoment erzeugt,
   gleichzeitiges Anregen mehrerer Schichten (S0, S1) des Volumenabschnitts mittels eines HF-Anregungspulses (41), welcher mit einer Repetitionszeit (TR) wiederholt wird, Variieren der Phase von zu erfassenden MR-Signalen einer selben der Schichten (S0, S1) von Repetitionszeit (TR) zu Repetitionszeit (TR), Anlegen eines Zusatz-Gradienten (50) in Schichtselektionsrichtung (SS) zusätzlich zu dem Schichtselektionsgradient (42), wobei der Zusatz-Gradient (50) ein Zusatz-Gradientenmoment erzeugt, welches über aufeinanderfolgende Repetitionszeiten (TR) konstant ist, wobei Gradientenmomente, welche ohne Berücksichtigung des Zusatz-Gradienten von der Gradientenecho-Sequenz erzeugt werden, entlang aller drei Raumrichtungen jeweils über die Repetitionszeit hinweg ausgeglichen sind, wobei das Zusatz-Gradientenmoment die Bedingung jeweils über die Repetitionszeit (TR) hinweg aufhebt, dass die Gradientenmomente der Gradientenecho-Sequenz entlang der Schichtselektionsrichtung (SS) ausgeglichen sind, und Erfassen der MR-Signale mittels eines Auslesegradienten (43).

2. Verfahren nach Anspruch 1,
**dadurch gekennzeichnet,**
**dass** der Zusatz-Gradient (50) während aller Repetitionszeiten (TR) konstant ist.

3. Verfahren nach Anspruch 2,
**dadurch gekennzeichnet,**
**dass** der Zusatz-Gradient (50) vor und/oder nach den HF-Anregungspulsen (41) geschaltet wird.

4. Verfahren nach Anspruch 3,
**dadurch gekennzeichnet,**
**dass** ein Verlauf des Zusatz-Gradienten (50) über aufeinanderfolgende Repetitionszeiten (TR) gleich ist.

5. Verfahren nach einem der vorhergehenden Ansprüche,
**dadurch gekennzeichnet,**
**dass** das Zusatz-Gradientenmoment abhängig von einem Abstand zwischen zumindest zwei der mehreren Schichten (S0, S1) und/oder abhängig von einer Phaseninkrement-Differenz der HF-Anregungspulse (41) der Schichten (S0, S1) bestimmt wird.

6. Verfahren nach Anspruch 5,
**dadurch gekennzeichnet,**
**dass** das Zusatz-Gradientenmoment DG gemäß folgender Gleichung (A1) bestimmt

$$DG = \frac{PD}{\gamma \cdot d} \quad \text{(A1)},$$

wobei PD der Phaseninkrement-Differenz der HF-Anregungspulse (41) der Schichten entspricht, wobei d dem Abstand der Schichten entspricht und wobei $\gamma$ dem gyromagnetischen Verhältnis entspricht.

7. Verfahren nach einem der vorhergehenden Ansprüche,
**dadurch gekennzeichnet,**
**dass** eine erste der Schichten (S0) einen Abstand von d0 in einer vorbestimmten Richtung von einem Isozentrum (33) des Grundmagnetfelds aufweist,
**dass** eine zweite der Schichten (S1) einen Abstand von d1 in der vorbestimmten Richtung vom Isozentrum (33) aufweist, dass der k-te HF-Anregungspuls der ersten Schicht (S0) eine Phase $P_0(k)$ aufweist, welche folgender Gleichung (A2) gehorcht

$$P_0(k) = -k * 90° - k * \Phi_G + \Phi_{C0} \quad \text{(A2)}$$

**dass** der k-te HF-Anregungspuls der zweiten Schicht (S1) eine Phase $P_1(k)$ aufweist, welche folgender Gleichung (A3) gehorcht

$$P_1(k) = +k * 90° - k * \Phi_G + \Phi_{C1} \quad \text{(A3)},$$

wobei $\Phi_{C0}$ einer konstanten Phase der ersten Schicht (S0) und $\Phi_{C1}$ einer konstanten Phase der zweiten Schicht (S1) entspricht,
wobei $\Phi_G$ einem Phaseninkrement entspricht, welches folgender Gleichung (4) genügt:

$$\Phi_G = 90° \cdot \frac{(d0 + d1)}{(d0 - d1)} \qquad (4),$$

und
wobei k bei 0 beginnt und über alle Zeilen der jeweiligen Schicht verläuft.

8. Verfahren nach Anspruch 7,
   **dadurch gekennzeichnet,**
   **dass** eine Phase $\Phi_E$ eines Empfängers (8, 8') der Magnetresonanzanlage (5) folgender Gleichung (5) gehorcht, um die MR-Signale zu erfassen:

$$\Phi_E = X(k) + k * \Phi0 - k * \Phi_G \qquad (5),$$

   wobei X(k) = 180° für ungerade k ist und sonst X(k) = 0° gilt.

9. Verfahren nach einem der vorhergehenden Ansprüche,
   **dadurch gekennzeichnet,**
   **dass** das Variieren der Phase der zu erfassenden MR-Signale für jede der gleichzeitig zu erfassenden Schichten (S0, S1) durchgeführt wird.

10. Verfahren nach einem der vorhergehenden Ansprüche,
    **dadurch gekennzeichnet,**
    **dass** das Variieren der Phase der zu erfassenden MR-Signale durchgeführt wird, indem ein weiterer Gradient vor und nach den HF-Anregungspulsen (41) in Schichtselektionsrichtung (SS) angelegt wird,
    **dass** das von dem weiteren Gradient erzeugte weitere Gradientenmoment vor dem jeweiligen HF-Anregungspuls (41) dem von dem weiteren Gradient erzeugten weiteren Gradientenmoment nach dem jeweiligen HF-Anregungspuls (41) entspricht, und dass das von dem weiteren Gradient erzeugte Gradientenmoment über aufeinanderfolgende Repetitionszeiten variiert.

11. Verfahren nach Anspruch 10,
    **dadurch gekennzeichnet,**
    **dass** das von dem weiteren Gradient erzeugte weitere Gradientenmoment in einer ersten Repetitionszeit dem negativen weiteren Gradientenmoment entspricht, welches von dem weiteren Gradient in einer zweiten Repetitionszeit, welche der ersten Repetitionszeit direkt folgt, erzeugt wird.

12. Verfahren nach einem der vorhergehenden Ansprüche,
    **dadurch gekennzeichnet,**
    **dass** das Variieren der Phase der zu erfassenden MR-Signale durchgeführt wird, indem die Phase der HF-Anregungspulse (41), welche eine selbe der Schichten (S0, S1) anregen, variiert wird.

13. Verfahren nach Anspruch 12,
    **dadurch gekennzeichnet,**
    **dass** für jede der Schichten (S0, S1) die Phase der HF-Anregungspulse (41) variiert wird.

14. Magnetresonanzanlage zum Erfassen von MR-Signalen eines Volumenabschnitts eines Untersuchungsobjekts (O) unter Einsatz einer Gradienten-Echosequenz, wobei die Magnetresonanzanlage (5) einen Grundfeldmagneten (1), ein Gradientenfeldsystem (3), mindestens eine HF-Antenne (4) und eine Steuereinrichtung (10) zur Ansteuerung des Gradientenfeldsystems (3) und der mindestens einen HF-Antenne (4), zum Empfang von von der mindestens einen HF-Antenne (4) aufgenommenen MR-Signalen (25) und zur Auswertung der MR-Signale umfasst, und wobei die Magnetresonanzanlage (5) derart ausgestaltet ist, dass die Magnetresonanzanlage (5) mit dem Gradientenfeldsystem (3) einen Schichtselektionsgradient (42) in Schichtselektionsrichtung (SS) schaltet, welcher ein ausgeglichenes Gradientenmoment erzeugt, mit der mindestens einen HF-Antenne (4) gleichzeitig mehrere Schichten (S0, S1) des Volumenabschnitts mittels eines HF-Anregungspulses (41), welcher mit einer Repetitionszeit (TR) wiederholt wird, anregt, mit der Steuereinrichtung (10) die Phase von zu erfassenden MR-Signalen einer selben der Schichten (S0, S1) von Repetitionszeit (TR) zu Repetitionszeit (TR) variiert, mit dem Gradientenfeldsystem (3) einen Zusatz-Gradient (50) in Schichtselektionsrichtung (SS) zusätzlich zu dem Schichtselektionsgradient (42) anlegt, wobei der Zusatz-Gradient (50) ein Zusatz-Gradientenmoment erzeugt, welches über aufeinanderfolgende Repetitionszeiten (TR) konstant ist, wobei Gradientenmomente, welche ohne Berücksichtigung des Zusatz-Gradienten von der Gradientenecho-Sequenz erzeugt werden, entlang aller drei Raumrichtungen jeweils über die Repetitionszeit hinweg ausgeglichen sind, wobei das Zusatz-Gradientenmoment jeweils über die Repetitionszeit hinweg die Bedingung aufhebt, dass die Gradientenmomente der Gradientenecho-Sequenz entlang der Schichtselektionsrichtung (SS) ausgeglichen sind, und wobei die Magnetresonanzanlage (5) derart ausgestaltet ist, dass die Magnetresonanzanlage (5) mit dem Gradientenfeldsystem (3) einen Auslesegradienten (43) schaltet und mittels des Auslesegradienten (43) und der mindestens einen HF-Antenne (4) die MR-Signale erfasst.

15. Magnetresonanzanlage nach Anspruch 14,
**dadurch gekennzeichnet,**
**dass** die Magnetresonanzanlage (5) zur Durchführung des Verfahrens nach einem der Ansprüche 2-13 ausgestaltet ist.

16. Computerprogrammprodukt, welches ein Programm umfasst und direkt in einen Speicher einer programmierbaren Steuereinrichtung (10) einer Magnetresonanzanlage (5) ladbar ist, mit Programm-Mitteln, um alle Schritte des Verfahrens nach einem der Ansprüche 1-13 auszuführen, wenn das Programm in der Steuereinrichtung (10) der Magnetresonanzanlage (5) ausgeführt wird.

17. Elektronisch lesbarer Datenträger mit darauf gespeicherten elektronisch lesbaren Steuerinformationen, welche derart ausgestaltet sind, dass sie bei Verwendung des Datenträgers (21) in einer Steuereinrichtung (10) einer Magnetresonanzanlage (5) das Verfahren nach einem der Ansprüche 1-13 durchführen.

**Claims**

1. Method for capturing MR signals of a predefined volume section (24) within an examination object (O) by means of a magnetic resonance installation (5) using a gradient echo sequence,
comprising steps as follows:

switching a layer selection gradient (42) in layer selection direction (LS), said layer selection gradient (42) producing a balanced gradient moment;
simultaneously exciting a plurality of layers (S0, S1) of the volume section by means of an HF excitation pulse (41) which is repeated according to a repetition time (TR);
varying from repetition time (TR) to repetition time (TR) the phase of MR signals to be captured from the same one of the layers (S0, S1);
establishing an additional gradient (50) in layer selection direction (LS) in addition to the layer selection gradient (42), wherein the additional gradient (50) produces an additional gradient moment which is constant over consecutive repetition times (TR), wherein gradient moments that are produced by the gradient echo sequence without taking into account the additional gradient are balanced along all three spatial directions over the repetition time in each case,
wherein the additional gradient moment, over the repetition time (TR) in each case, overrides the condition that the gradient moments of the gradient echo sequence are balanced along the layer selection direction (LS); and
capturing the MR signals by means of a readout gradient (43).

2. Method according to claim 1,
**characterised in that**
the additional gradient (50) is constant during all repetition times (TR).

3. Method according to claim 2,
**characterised in that**
the additional gradient (50) is switched before and/or after the HF excitation pulses (41).

4. Method according to claim 3,
**characterised in that**
a profile of the additional gradient (50) is identical over consecutive repetition times (TR).

**5.** Method according to one of the preceding claims, **characterised in that** the additional gradient moment is determined as a function of a distance between at least two of the plurality of layers (S0, S1) and/or as a function of a difference in phase increment of the HF excitation pulses (41) of the layers (S0, S1).

**6.** Method according to claim 5, **characterised in that** the additional gradient moment DG is determined according to the following equation (A1):

$$DG = \frac{PD}{\gamma \cdot d} \quad \text{(A1)},$$

wherein PD corresponds to the difference in phase increment of the HF excitation pulses (41) of the layers, wherein d corresponds to the distance of the layers, and wherein $\gamma$ corresponds to the gyromagnetic ratio.

**7.** Method according to one of the preceding claims, **characterised in that** a first of the layers (S0) has a distance of d0 in a predefined direction from an isocentre (33) of the basic magnetic field, **in that** a second of the layers (S1) has a distance of d1 in the predefined direction from the isocentre (33), **in that** the $k^{th}$ HF excitation pulse of the first layer (S0) has a phase $P_0(k)$ which obeys the following equation (A2):

$$P_0(k) = -k * 90° - k * \Phi_G + \Phi_{C0} \quad \text{(A2)},$$

in that the $k^{th}$ HF excitation pulse of the second layer (S1) has a phase $P_1(k)$ which obeys the following equation (A3):

$$P_1(k) = +k * 90° - k * \Phi_G + \Phi_{C1} \quad \text{(A3)},$$

wherein $\Phi_{C0}$ corresponds to a constant phase of the first layer (S0) and $\Phi_{C1}$ corresponds to a constant phase of the second layer (S1), wherein $\Phi_G$ corresponds to a phase increment which satisfies the following equation (A4):

$$\Phi_G = 90° \cdot \frac{(d0 + d1)}{(d0 - d1)} \quad \text{(A4)},$$

and wherein k starts at 0 and runs over all rows of the respective layer.

**8.** Method according to claim 7, **characterised in that** a phase $\Phi_E$ of a receiver (8, 8') of the magnetic resonance installation (5) obeys the following equation (A5) in order to capture the MR signals:

$$\Phi_E = X(k) + k * \Phi0 - k * \Phi_G \quad \text{(A5)},$$

wherein X(k) = 180° if k is an odd number and otherwise it applies that X(k) = 0°.

**9.** Method according to one of the preceding claims, **characterised in that**

the varying of the phase of the MR signals to be captured is performed for each of the layers to be captured simultaneously (S0, S1).

10. Method according to one of the preceding claims,
    **characterised in that**
    the varying of the phase of the MR signals to be captured is performed by establishing a further gradient before and after the HF excitation pulses (41) in layer selection direction (LS), **in that**
    the further gradient moment produced by the further gradient before the respective HF excitation pulse (41) corresponds to the further gradient moment produced by the further gradient after the respective HF excitation pulse (41), and **in that** the gradient moment produced by the further gradient varies over consecutive repetition times.

11. Method according to claim 10,
    **characterised in that**
    the further gradient moment produced by the further gradient in a first repetition time corresponds to the negative further gradient moment which is produced by the further gradient in a second repetition time which directly follows the first repetition time.

12. Method according to one of the preceding claims,
    **characterised in that**
    the varying of the phase of the MR signals to be captured is performed by means of varying the phase of the HF excitation pulses (41) which excite the same one of the layers (S0, S1).

13. Method according to claim 12,
    **characterised in that**
    the phase of the HF excitation pulses (41) is varied for each of the layers (S0, S1).

14. Magnetic resonance installation for capturing MR signals of a volume section of an examination object (O) using a gradient echo sequence,
    wherein the magnetic resonance installation (5) comprises a basic field magnet (1), a gradient field system (3), at least one HF antenna (4) and a control entity (10) for activating the gradient field system (3) and the at least one HF antenna (4), for receiving MR signals (25) picked up by the at least one HF antenna (4), and for evaluating the MR signals, and wherein the magnetic resonance installation (5) is configured such that the magnetic resonance installation (5) uses the gradient field system (3) to switch a layer selection gradient (42) in layer selection direction (LS), said layer selection gradient (42) producing a balanced gradient moment; uses the at least one HF antenna (4) to simultaneously excite a plurality of layers (S0, S1) of the volume section by means of an HF excitation pulse (41) which is repeated according to a repetition time (TR); uses the control entity (10) to vary from repetition time (TR) to repetition time (TR) the phase of MR signals to be captured from the same one of the layers (S0, S1); uses the gradient field system (3) to establish an additional gradient (50) in layer selection direction (LS) in addition to the layer selection gradient (42), wherein the additional gradient (50) produces an additional gradient moment which is constant over consecutive repetition times (TR), wherein gradient moments that are produced by the gradient echo sequence without taking into account the additional gradient (50) are balanced along all three spatial directions over the repetition time in each case,
    wherein the additional gradient moment, over the repetition time in each case, overrides the condition that the gradient moments of the gradient echo sequence are balanced along the layer selection direction (LS) and
    wherein the magnetic resonance installation (5) is embodied such that the magnetic resonance installation (5) uses the gradient field system (3) to switch a readout gradient (43) and capture the MR signals by means of the readout gradient (43) and the at least one HF antenna (4).

15. Magnetic resonance installation according to claim 14,
    **characterised in that**
    the magnetic resonance installation (5) is so configured as to perform the method according to one of claims 2 to 13.

16. Computer program product which comprises a program and can be loaded directly into a memory of a programmable control entity (10) of a magnetic resonance installation (5), with program resources, in order to execute all steps of the method according to one of claims 1 to 13 when the program is executed in the control entity (10) of the magnetic resonance installation (5).

17. Electronically readable data medium, on which is stored electronically readable control information that is so configured as to perform the method according to one of claims 1 to 13 when the data medium (21) is used in a control entity (10) of a magnetic resonance installation (5).

**Revendications**

1. Procédé de détection de signaux RM d'une partie (24) en volume déterminée à l'avance au sein d'un objet (O) à examiner au moyen d'une installation (5) de résonnance magnétique en utilisant une séquence d'écho de gradient, comprenant les stades suivants :

   mise d'un gradient (42) de sélection de couche dans une direction (SS) de sélection de couche, qui produit un moment de gradient compensé,
   excitation simultanée de plusieurs couches (S0, S1) de la partie de volume au moyen d'une impulsion (41) d'excitation HF, qui est répétée à un temps (TR) de répétition,
   variation des phases de signaux RM à détecter d'une même des couches (S0, S1) d'un temps TR de répétition à un temps TR de répétition,
   application d'un gradient (50) supplémentaire dans la direction (SS) de sélection de couche en plus du gradient (42) de sélection de couche, le gradient (50) supplémentaire produisant un moment de gradient supplémentaire, qui est constant sur des temps (TR) de répétition successifs, des moments de gradient qui sont produits sans tenir compte du gradient supplémentaire par la séquence d'écho de gradient étant compensés suivant toutes les trois directions de l'espace respectivement au-delà du temps de répétition, le moment de gradient supplémentaire levant respectivement au-delà du temps (TR) de répétition la condition que les moments de gradient de la séquence d'écho de gradient soient compensés dans la direction (SS) de sélection de couche et détection des signaux RM au moyen d'un gradient (43) de lecture.

2. Procédé suivant la revendication 1,
   **caractérisé en ce que**
   le gradient (50) supplémentaire est constant pendant tous les temps (TR) de répétition.

3. Procédé suivant la revendication 2,
   **caractérisé**
   **en ce qu'**on met le gradient (50) supplémentaire avant et/ou après les impulsions (41) d'excitation HF.

4. Procédé suivant la revendication 3,
   **caractérisé**
   **en ce qu'**une courbe du gradient (50) supplémentaire sur des temps (TR) de répétition successifs est pareille.

5. Procédé suivant l'une des revendications précédentes, **caractérisé**
   **en ce qu'**on détermine le moment de gradient supplémentaire en fonction d'une distance entre au moins deux parmi les plusieurs couches (S0, S1) et/ou en fonction d'une différence d'incrément de phase de l'impulsion (41) d'excitation HF des couches (S0, S1).

6. Procédé suivant la revendication 5,
   **caractérisé**
   **en ce que** l'on détermine le moment DG de gradient supplémentaire par l'équation (A1) suivante :

$$DG = \frac{PD}{\gamma.d} \ (A1)$$

   dans laquelle PD est la différence d'incrément de phase de l'impulsion (41) d'excitation HF des couches, d étant la distance entre les couches et $\gamma$ étant le rapport gyromagnétique.

7. Procédé suivant l'une des revendications précédentes, **caractérisé**
   **en ce qu'**une première des couches (S0) a une distance de d0 dans une direction déterminée à l'avance à un isocentre (33) du champ magnétique de base,
   **en ce qu'**une deuxième des couches (S1) a une distance de d1 dans la direction déterminée à l'avance à l'isocentre (33),

**en ce que** la kième impulsion d'excitation HF de la première couche (S0) a une phase $P_0(k)$, qui obéit à l'équation (A2) suivante :

$$P_0(k) = -k * 90° - k * \Phi_G + \Phi_{C0} \quad (A2)$$

en ce que la kième impulsion d'excitation HF de la deuxième couche (S1) a une phase $P_1(K)$, qui obéit à l'équation (A3) suivante :

$$P_1(k) = +k * 90° - k * \Phi_G + \Phi_{C1} \quad (A3)$$

dans lesquelles $\Phi_{C0}$ correspond à une phase constante de la première couche (S0) et $\Phi_{C1}$ correspond à une phase constante de la deuxième couche (S1)

dans lesquelles $\Phi_G$ correspond à un incrément de phase, qui satisfait l'équation (4) suivante :

$$\Phi_C = 90° \frac{(d0 + d1)}{(d0 - d1)} \quad (4)$$

et

dans lesquelles k commence à 0 et s'étend sur toutes les lignes de la couche respective.

**8.** Procédé suivant la revendication 7, **caractérisé**

en ce qu'une phase $\Phi_E$ d'un récepteur (8, 8') de l'installation (5) de résonnance magnétique obéit à l'équation (5) suivante, pour détecter les signaux RM :

$$\Phi_E = X(k) + k * \Phi 0 - k * \Phi_G \quad (5)$$

dans laquelle X(k) = 180° pour k impair et on a sinon X(k) = 0°

**9.** Procédé suivant l'une des revendications précédentes, **caractérisé**

**en ce que** l'on effectue la variation de phase des signaux RM à détecter pour chacune des couches (S0, S1) à détecter simultanément.

**10.** Procédé suivant l'une des revendications précédentes, **caractérisé**

**en ce qu'**on effectue la variation de phase des signaux RM à détecter en appliquant un autre gradient avant et après les impulsions (41) d'excitation HF dans la direction (SS) de sélection de couche,

**en ce que** l'autre moment de gradient produit par l'autre gradient avant l'impulsion (41) d'excitation HF respective correspond à l'autre moment de gradient produit par l'autre gradient après l'impulsion d'excitation (41) HF respective et en ce que le moment de gradient produit par l'autre gradient varie sur les temps de répétition successifs.

**11.** Procédé suivant la revendication 10, caractérisé

en ce l'autre moment de gradient produit par l'autre gradient dans un premier temps de répétition correspond à l'autre moment de gradient négatif, qui est produit par l'autre gradient dans un deuxième temps de répétition qui suit immédiatement le premier temps de répétition.

**12.** Procédé suivant l'une des revendications précédentes, **caractérisé**

**en ce qu'**on effectue la variation de phase des signaux RM à détecter en faisant varier les phases de l'impulsion (41) d'excitation HF, qui excite une même des couches (S0, S1).

**13.** Procédé suivant la revendication 12, caractérisé

**en ce qu'**on fait varier pour chacune des couches (S0, S1) la phase de l'impulsion (41) d'excitation HF.

**14.** Installation de résonnance magnétique pour la détection de signaux RM d'une partie de volume d'un objet (O) à examiner en utilisant une séquence d'écho de gradient,

dans laquelle l'installation (5) de résonnance magnétique comprend un aimant (1) de champ de base, un système (3) de champ de gradient, au moins une antenne (4) HF et un dispositif (10) de commande pour la commande du système (3) de champ de gradient et de la au moins une antenne (4) HF pour la réception de signaux (25) RM reçus par la au moins une antenne (4) HF et pour l'exploitation des signaux RM et

dans laquelle l'installation (5) de résonnance magnétique est conformée de manière à ce que l'installation (5) de résonnance magnétique mette avec le système (3) de champ de gradient un gradient (42) de sélection de couche dans la direction (SS) de sélection de couche, qui produit un moment de gradient compensé de manière à exciter par la au moins une antenne (4) HF en même temps plusieurs couches (S0, S1) de la partie de volume au moyen d'une impulsion (41) d'excitation HF, qui est répétée à un temps (TR) de répétition de manière à faire varier par le dispositif (10) de commande la phase de signaux RM à détecter d'une même des couches (S0, S1) d'un temps (TR) de répétition à un temps (TR) de répétition de manière à appliquer par le système (3) de champ de gradient un gradient (50) supplémentaire dans la direction (SS) de sélection de couche supplémentairement au gradient (42) de sélection de couche, le couche (50) supplémentaire produisant un moment de gradient supplémentaire qui est constant sur des temps (TR) de répétition successif, des moments de gradient qui sont produits sans tenir compte du gradient supplémentaire par la séquence d'écho de gradient étant compensés dans toutes les trois directions de l'espace respectivement au-delà du temps de répétition, le moment de gradient supplémentaire levant respectivement au-delà du temps de répétition la condition que les moments de gradient de la séquence d'écho de gradient soient compensés dans la direction (SS) de sélection de couche et l'installation (5) de résonnance magnétique étant conformée de manière à ce que l'installation (5) de résonnance magnétique mette avec le système (3) de champ de gradient un gradient (43) de lecture et détecte les signaux RM au moyen du gradient (43) de lecture et de la au moins une antenne (4) HF.

**15.** Installation de résonnance magnétique suivant la revendication 14,
**caractérisée**
**en ce que** l'installation (5) de résonnance magnétique est conformée pour effectuer le procédé suivant l'une des revendications 2 à 13.

**16.** Produit de programme d'ordinateur, qui comprend un programme et qui peut être chargé directement dans une mémoire d'un dispositif (10) de commande programmable d'une installation (5) de résonnance magnétique, ayant des moyens de programme pour effectuer tous les stades du procédé suivant l'une des revendications 1 à 13 lorsque le programme est exécuté dans le dispositif (10) de commande de l'installation (5) de résonnance magnétique.

**17.** Support de données déchiffrable électroniquement, sur lequel sont mises en mémoire des informations de commande déchiffrables électroniquement et qui sont conformées de manière à ce qu'elles exécutent, lors de l'utilisation du support (21) de données dans un dispositif (10) de commande d'une installation (5) de résonnance magnétique, le procédé suivant l'une des revendications 1 à 13.

FIG 1

FIG 2

S0

S1

d0

d1

33

32

34

90°

0°

-90°

Δω

32

FIG 3

EP 3 176 596 B1

FIG 4

HF

SS

AL

PK

ADC

41 41 41 41

42b 42b 42b 42b
42a 42c 42a 42c 42a 42c 42a 42c
50a 50b 43b 50a 43b 50a 43b 43b 50a
50b 50b 50b 50b
43a 43c 43a 43c 43a 43c 43a

44b 44b 44b 44a
44a 44a 44b

0° 45 180° 45 0° 45

TR TR TR

EP 3 176 596 B1

FIG 5

FIG 6

$\Phi_G$

$\Delta\omega$

$-90°$   $0°$   $90°$

34   35

d0   d1

33

S0

S1

EP 3 176 596 B1

FIG 7

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102014202358 A1

- US 20130271128 A1

**In der Beschreibung aufgeführte Nicht-Patentliteratur**

- **F.A. BREUER U.A.** Controlled Aliasing in Parallel Imaging Results in Higher Acceleration (CAIPIRINHA) for Multi-Slice Imaging. *Magn. Reson. Med.,* 2005, vol. 53 (3), 684-691

- **D. STÄB U.A.** CAIPIRINHA accelerated SSFP imaging. *Magn. Reson. Med.,* 2011, vol. 65, 157-164